# EUROPEAN PATENT SPECIFICATION

(11) **EP 0 880 894 B1**
(45) Date of publication and mention of the grant of the patent: **17.04.2002**
(21) Application number: 98109583.9
(22) Date of filing: 26.05.1998
(51) Int. Cl.: A01N 65/00

(54) **Selective bactericide against food-borne pathogenic bacteria**
Selektives Bakterizid zur Hemmung pathogener Bakterien aus Nahrungsmitteln
Bactéricide sélectif pour l'inhibition des pathogènes bactériens d'origine alimentaire

(30) Priority: 27.05.1997 JP 15171197; 25.11.1997 JP 33828197
(43) Date of publication of application: 02.12.1998
(73) Proprietor: Sato, Naohiko, Chofu-shi, Tokyo-to (JP)
(72) Inventor: Tomita, Toshio, Sendai-shi, Miyagi-ken (JP); Kamio, Yoshiyuki, Sendai-shi, Miyagi-ken (JP); Sato, Naohiko, Chofu-shi, Tokyo (JP)
(74) Representative: Prüfer, Lutz H., Dipl.-Phys.

(56) References cited:
- EP-A- 0 525 740
- US-A- 4 647 458
- DATABASE WPI Section Ch, Week 7629 Derwent Publications Ltd., London, GB; Class D13, AN 76-55093X XP002077200 & JP 51 063 953 A (MORITA KAGAKU KOGYO KK)
- DATABASE WPI Section Ch, Week 7622 Derwent Publications Ltd., London, GB; Class B04, AN 76-41037X XP002077201 & JP 51 044 616 A (KOSHIRO CHUJI SHOTE)
- DATABASE WPI Section Ch, Week 8035 Derwent Publications Ltd., London, GB; Class B04, AN 80-61123C XP002077202 & JP 55 092 323 A (OSHIRO CHUJI SHOTEN)
- DATABASE WPI Section Ch, Week 9145 Derwent Publications Ltd., London, GB; Class C04, AN 91-329180 XP002077203 & JP 03 220 109 A (SEIN T)
- DATABASE WPI Section Ch, Week 8017 Derwent Publications Ltd., London, GB; Class D13, AN 80-30046C XP002077204 & JP 55 035 023 A (YAMAGUCHI T)

## Description

### FIELD OF THE INVENTION

The present invention relates to a Stevia-derived selective bactericide against food-borne pathogenic bacteria, and more particularly to a bactericide having bactericidal activity towards food-borne pathogenic bacteria including Escherichia coli O 157, and Salmonella and Staphylococcus aureus, but having no bactericidal activity towards intestinal bacteria such as Bifidobacteria and Lactobacilli which are useful for human bodies and animals.

### BACKGROUND OF THE INVENTION

In recent years, the habit of eating various foods raw has been established even in the summer season of high temperature and high humidity which promotes the proliferation of bacteria. On the other hand, antibiotics are used in large amounts with the development of various antibiotics, because even somewhat serious food poisoning is readily mitigated thereby. As a result, resistant strains appear, resulting in introduction of serious problems. In the history of antibiotics, competitions between the development of novel antibiotics and the appearance of resistant strains have been repeated.

On the other hand, Stevia is known as a raw material for a natural low-caloric sweetener. Steviocide or levaudiocide contained in leaves of Stevia in large amounts shows strong sweetness even in small amounts, so that it is used as a low-caloric natural sweetener instead of sugar.

Japanese Examined Patent Publication No. 7-13021 discloses that a concentrated fermented liquid extracted from stems and leaves of Stevia is effective as a therapeutic oral liquid medicine for alimentary diseases. In this technique, the mature stems of Stevia is used as a raw material, and a dried powder thereof is boiled in and extracted with water. Then, the extract is concentrated, and fermented at 25 °C over half a year or longer. The specification describes that this fermented liquid is practically effective against constipation, loss of appetite, sensitivity to cold and epigastric distress.

JP-A-51 063 953 discloses pharmacologically active substances from leaves of Stevia rebaudiana bertoni having antibacterial activities.

In JP-A-51 044 616 a fractionated extract of Stevia rebaudiana bertoni is disclosed, having an anti-bacterial property against gram-negative bacteria.

In JP-A-55 092 323 an antibacterial substance obtained from (extracts of) Stevia rebaudiana bertoni is described, which is employed togehter with other antibacterial substances, to increase the preserving effect on food.

JP-A-3 220 109 discloses a fermented extract of Stevia rebaudiana comprising organic acids which is used in horticultural fertilisers for producing vegetables and fruits having improved natural sweetness.

The technique described in Japanese Examined Patent Publication No. 7-13021 is effective against general disorders of the digestive organs. However it is not disclosed that these liquid extracts are effective against food-borne pathogenic bacteria. It is especially not disclosed that fermented extracts from Stevia hav a selective bactericidal activity against pathogenic bacteria such as E. coli O 157.

In recent years, alimentary diseases caused by E. coli O 157 are spreading throughout industrial nations. Antibiotics can not be easily used because E. coli itself is a bacterium which is usually present in the human body. In addition, the verocytotoxin produced by E. coli O 157 has also strong toxicity. Conventional antibiotics usually lead to the appearance of resistant strains. Accordingly other substances which are effective against food poisoning are desired which may be derived from natural products and can be used alone or in combination with antibiotics.

It is the object of the invention to provide an improved bactericide against food-borne bacteria and a new use for fermented extracts from plant tissue of Stevia.

The object is solved according to the independent claims. The dependent claims relate to preferred embodiments.

### SUMMARY OF THE INVENTION

It has been discovered that non-fermented or fermented extracts from plant tissue of Stevia contain substances which have a selective bactericidal activity against food-borne pathogenic bacteria and no adverse effect on the intestinal bacterial flora and that this selective bactericidal activity is remarkably increased by adding an organic acid to the extract. The thus formed selective bactericide, which shows a strong bactericidal activity towards food-borne pathogenic bacteria, also inhibits the production of verocytotoxin. It especially shows no bactericidal activity against Lactobacilli and Bifidobacteria.

Active ingredients contained in the plant tissue of Stevia have not been identified yet. However, the ingredients are presumed to be a plurality of hydrophilic substances having relatively low molecular weights because the components are readily extracted with cold water, hot water or a water-miscible solvent.

### BRIEF DESCRIPTION OF THE DRAWINGS

Fig. 1 is a graph showing relations between the concentration of an aged Stevia extract original fluid and the bactericidal effect thereof on pathogenic E. coli;
Fig. 2 is a graph showing a relation between the concentration of the Stevia extract original fluid and the amount of verocytotoxin produced by E. coli EC183;
Fig. 3 is a graph showing a relation between the concentration of the Stevia extract original fluid and the amount of verocytotoxin produced by E. coli EC164;
Fig. 4 is a graph showing a bactericidal effect of the aged Stevia extract original fluid on other pathogenic microorganisms;
Fig. 5 is a graph showing the pH dependence of an effect of the aged Stevia extract original fluid on E. coli EC164;
Fig. 6 is a graph comparing a bactericidal effect of single organic acids with that of mixtures of the Stevia extract original fluid and the organic acids on E. coli EC164;
Fig. 7 is a graph showing a bactericidal effect of acetic acid and that of a mixture of non-aged Stevia extract and acetic acid on E. coli EC164; and
Fig. 8 is a graph showing a bactericidal effect of the aged Stevia extract on Bifidobacteria and Lactobacilli.

### DETAILED DESCRIPTION OF THE INVENTION

Stevia used as a raw material in the present invention includes a perennial composite plant of South America origin, Stevia rebaudiana Bertoni and related plants thereof. Results of experiments indicate that the active ingredients are contained in large amounts in leaves and stems thereof, particularly in stems before putting forth buds and stems of mature plants, and also in roots, blossoms and juvenile plants. Steviocide and levaudiocide known as sweetening ingredients of Stevia are found to have no bactericidal activity. Accordingly, the object of the present invention can be achieved as long as the extract from the whole plant tissue of Stevia is contained. The extract may be blended with another substance.

There is no particular limitation on extracting methods, as long as the ingredients contained in the plant tissue of Stevia can be extracted. Examples of such methods include extraction with hot water, extraction with cold water and extraction with a lower alcohol. Although the active ingredients have not been identified yet, they are considered to be hydrophilic substances. They are therefore extracted with a lower alcohol such as methyl alcohol, ethyl alcohol, n-propyl alcohol or isopropyl alcohol.

Prior to the extraction, only the leaves are picked off or terrestrial portions of the plants are cut down, and pulverized as such or after drying. Then, the extraction is carried out. They may also be pulverized and extracted immediately after reaping. For improving the extraction efficiency, it is preferred that they are cut and further pulverized. In general, roots are not used, for germination expected in next spring.

The extraction is achieved by boiling the powder in water for 30 minutes or longer. In some cases, a residue separated from a first extract is extracted again with fresh hot water, thereby being able to extract the active ingredients almost completely.

Further, the active ingredients are also eluted in sufficient amounts in a supernatant solution obtained by immersing the fine powder in water at ordinary temperature for 2 or 3 days or at 30°C to 50°C for 3 hours.

The Stevia extract thus obtained is concentrated and used as an extract original fluid having a solid concentration of 16 to 20 W/V%. In general, 0.3 to 3 liters of the extract original fluid is obtained from 1 kg of fine powder of Stevia.

Remarkable selective bactericidal activity is developed by adding an organic acid to the extract original fluid. Examples of such organic acids include acetic acid, lactic acid, propionic acid, valeric acid, citric acid, tartaric acid, malic acid, maleic acid, fumaric acid and acrylic acid. Inorganic acids such as hydrochloric acid can not be expected to have the effect of promoting bactericidal activity.

Storage of the extract original fluid in a vessel at ordinary temperature results in fermentation to fill the vessel with carbon dioxide gas. It is therefore preferred that the extract original fluid is aged in a vessel equipped with a device automatically releasing carbon dioxide gas when the inner pressure reaches a specified value. Although the generation of carbon dioxide gas is significant at an initial stage of the aging and is gradually depressed, the fermentation continues for at least 5 years. The formation of acetic acid, lactic acid and other organic acids by the aging gives bactericidal activity to the aged extract. The addition of an organic acid such as acetic acid or lactic acid to this aged extract leads to a marked improvement in bactericidal activity.

Further, the addition of an organic acid such as acetic acid or lactic acid to the extract immediately after extraction also provides excellent bactericidal activity.

The extract from the stems and leaves of Stevia is neutral and shows no bactericidal activity towards the food-borne pathogenic bacteria. It has been found that, when the extract is allowed to stand at ordinary temperature, carbon dioxide gas is generated by fermentation and organic acids such as acetic acid and lactic acid are formed at the same time. The bactericidal activity is extremely increased by the addition of such an organic acid to the fermented extract or the extract immediately after extraction. Moreover, this bactericidal activity is selective, and causes no substantial harm to the growth of useful bacteria which keep microorganism floras in the digestive organs of animals normal, such as Lactobacilli and Bifidobacteria. Accordingly, even if this bactericide is orally administered, it has no adverse effect on the bacterial floras in the digestive organs, but selectively acts on alimentary pathogenic microorganism floras of humans and animals, particularly to E. coli O 157, to inhibit the proliferation thereof and further also the secretion of verocytotoxin.

Further, the bactericide can be added to feed for domestic animals which is liable to become the source of infection, thereby preventing the domestic animals from being infected with the food-borne pathogenic bacteria.

### EXAMPLES

### (1) Preparation of Stevia Test Extract

Seedlings of Stevia were planted in the spring, and terrestrial portions thereof were cut down before putting forth buds. The harvest was air-dried after reaping, and the leaves were separated from the stems. The leaves were pulverized to a size of 10 µm or less with a grinder to produce Stevia leave powder. The stems were cut to a length of 5 cm and then pulverized to a size of 10 µm or less with a grinder to produce Stevia stem powder.

The Stevia leave powder and Stevia stem powder thus produced were blended at a ratio of 3:7 and uniformly mixed to prepare mixed Stevia powder to produce Stevia extract.

The mixed Stevia powder was boiled in 10 liters of water based on 1 kg of powder for 1 hour. After cooling, strained lees were separated with a wringer, and the extract was boiled down for 3 hours to obtain 1.2 liters of a non-aged Stevia extract original fluid. This non-aged extract original fluid was allowed to stand in a vessel at ordinary temperature. As a result, the fermentation proceeded and carbon dioxide gas was required to be intermittently released.

The fermentation violently occurred at the initial stage, and gradually settled down after an elapse of 3 to 4 months. However, the fermentation continued after an elapse of one year, or even after an elapse of two or more years.

In the examples, the Stevia extract original liquid fermented for one year was used. Accordingly, the Stevia extract used in the examples corresponds to 0.83 g/cm³ of raw mixed Stevia powder. Further, this Stevia extract had a solid concentration of about 20%.

### (2) Strains

The food-borne pathogenic bacteria used for test were the following strains which had been clinically isolated and preserved at Miyagi Prefectural Institute of Public Health and Environment.

Unless otherwise stated, the bacteria were precultured statically in brain-heart infusion broth overnight, and 0.5 ml of the preculture was then transferred to 5 ml of fresh broth and cultivated at 37 °C for 3 hours with gentle stirring.

### (3) Assay of Bactericidal Activity

Bactericidal activity was assayed according to the following procedure.

Approximately 1.0×10⁹ colony-forming units (cfu) of bacteria were suspended in 0.5 ml of the brain-heart infusion broth, mixed with 0.5 ml of a mixture of the aged Stevia extract original liquid and physiological saline, and statically cultivated at 37°C for 2 hours. Each mixture was prepared by serial 10-fold dilution. Then, 0.1-ml aliquots of the dilutions were withdrawn, plated onto normal agar plates, and allowed to stand at 37 °C for 2 hours, followed by dilution with physiological saline. Specified quantities of the dilutions were plated onto normal agar plates and cultivated to assay colony-forming units of bacteria.

### EXAMPLE 1

The bactericidal activity of Stevia extract was tested in respect to the following enterohemorrhagic E. coli and pathogenic E. coli.
E. coli
EC282 (O 157:H7, VT1-producing)
EC177 (O 157:H7, VT2-producing)
EC183 (O 157:H7, VT1- and VT2-producing)
EC164 (O 157:H7, VT1- and VT2-producing)
EC189 (O 157:H⁻ VT1- and VT2-producing)
EC217 (O 26:H11, VT1-producing)
EC300 (O 114:H⁻ VT2-producing)
EC174 (O 124:H⁻ enteroinvasive E. coli)
EC127 (O 169:H41, heat-stable enterotoxin-producing E. coli)

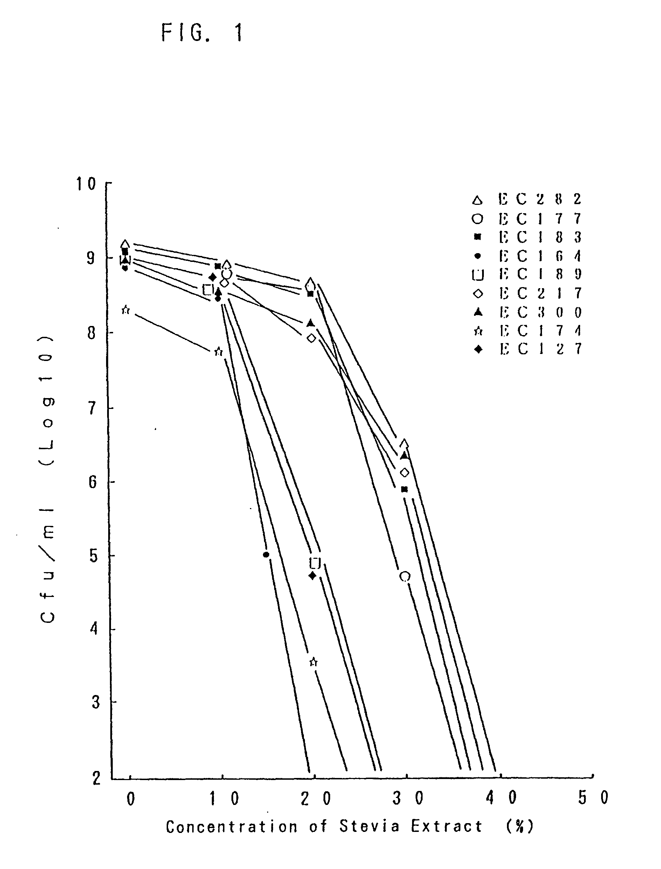

Fig. 1 is a graph showing relations between the concentration of the Stevia extract original fluid and the bactericidal effect on the pathogenic E. coli.

As is apparent from Fig. 1, each strain was reduced from approximately 10⁹ cfu/ml to 10² cfu/ml or less when incubated in a medium containing the aged Stevia extract original fluid at a concentration of 40 V/V% or more at 37 °C for 2 hours. Although there were some relatively resistant strains, they were also efficiently killed by a medium containing the Stevia extract original fluid at a concentration of 30 V/V% or more.

The pathogenic E. coli strains tested were apparently divided into two groups with regard to their susceptibility to the aged Stevia extract original fluid. Enterohemorrhagic E. coli EC164 and EC189, enteroinvasive E. coli EC174 and toxinogenic E. coli EC127 were very sensitive strains, which were decreased from 10⁹ cfu/ml to 10⁵ cfu/ml or less by incubation in a medium containing the Stevia extract original fluid at a concentration of 20 V/V% at 37°C for 2 hours. On the other hand, enterohemorrhagic E. coli EC183, EC282, EC177 and EC300 were relatively resistant strains, which could not be substantially killed under equivalent conditions.

### EXAMPLE 2

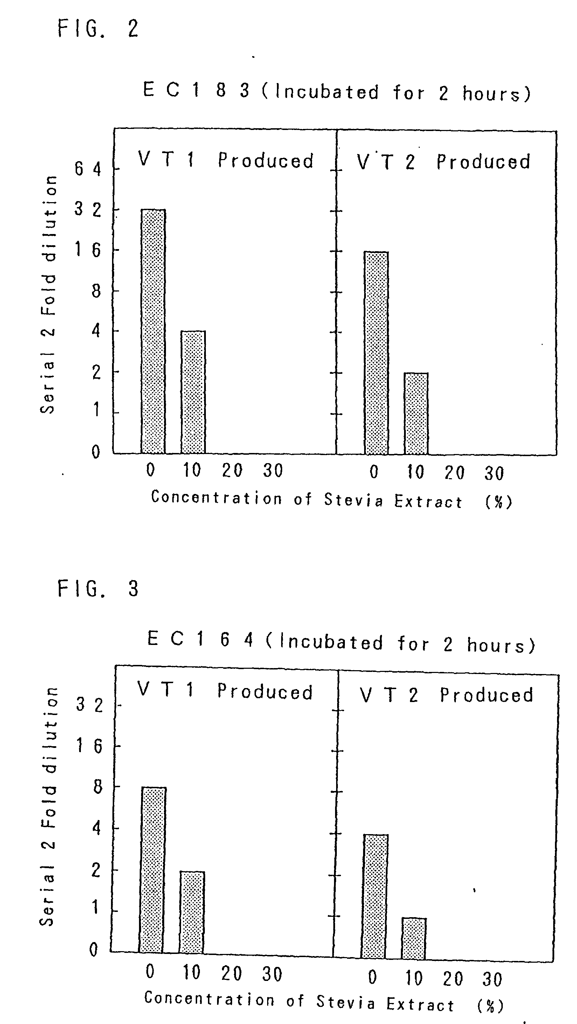

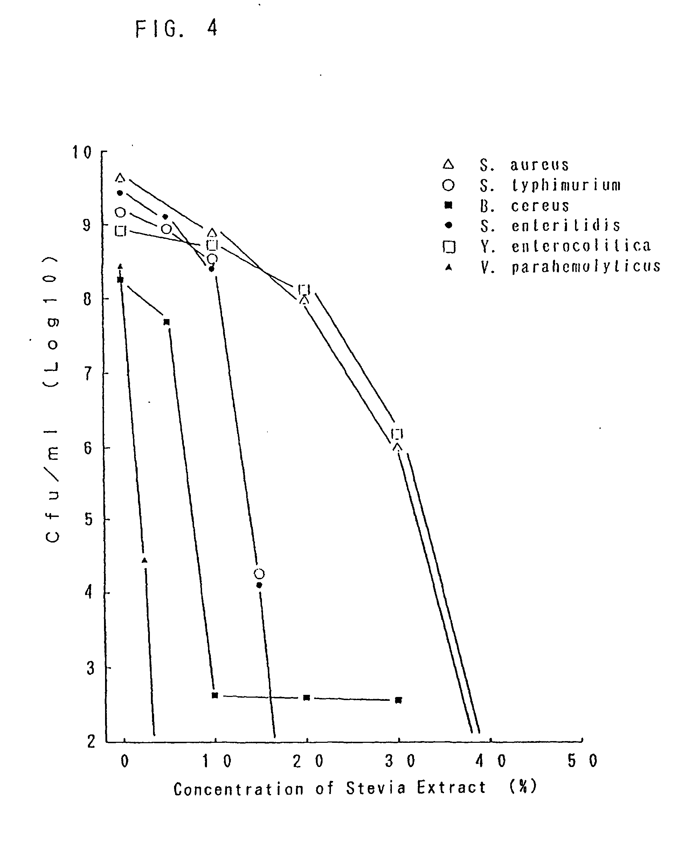

A sensitive E. coli strain (EC164 strain) and a relatively resistant E. coli strain (EC183 strain) were incubated in the presence of the aged Stevia extract original fluid at 37°C for 2 hours, followed by centrifugation. Then, verocytotoxin 1 (VT1) and verocytotoxin 2 (VT2) in the supernatants were assayed. Results thereof are shown in Figs. 2 and 3.

As is apparent from Figs. 2 and 3, it became clear that the aged Stevia extract original fluid inhibited the production and/or secretion of VT1 and VT2 by the E. coli strains even at concentrations of 10 and 20 V/V%. As is apparent from Fig. 1, these concentrations was too low to sufficiently develop bactericidal activity. Actually, when the concentration of the Stevia extract original fluid is 10 V/V%, the number of colonies was large, but each colony was reduced in size. The ability of producing verocytotoxin by E. coli was assayed according to the following procedure. Enterohemorrhagic E. coli was incubated statically overnight in 5 ml of CA-YE medium. E. coli cells were collected by centrifugation at 1500 g for 15 minutes, washed twice by centrifugation, and resuspended in CA-YE medium. The E. coli suspension was mixed with the same volume of the above-mentioned aged Stevia extract original fluid plus physiological saline, incubated at 37°C for 2 hours, and centrifuged at 1500 g for 15 minutes. The supernatants obtained by centrifugation were serially 2-fold diluted, and verocytotoxin contained in the supernatants was immunologically assayed by use of "Verocytotoxin Detecting Kit" (manufactured by Denka Seiken Co.) according to reversed passive latex agglutination. That is to say, when the supernatants of bacteria incubated are allowed to react with latex beads to which anti-VT1 or VT2 antibodies are bound, VT1 or VT2 in the supernatants binds to the antibodies on the beads to flocculate.

### EXAMPLE 3

The bactericidal effect of the Stevia extract original fluid on other pathogenic bacteria was assayed. The bacteria used are as follows:

| Salmonella enteritidis | |
|---|---|
| SA188 | |
| SA181 | |

| Salmonella typhimurium | |
|---|---|
| SA173 | |

| Staphylococcus aureus | |
|---|---|
| ST86 coagulase type II | |
| ST116 coagulase type III | |
| ST126 coagulase type VII | |
| ST82 coagulase type VIII | |

| Bacillus cereus | |
|---|---|
| BC43 | |
| BC37 | |

| Yersinia enterocolitica | |
|---|---|
| YE21 | 03 |
| YE15 | 05 |

| Vibrio parahemolyticus | |
|---|---|
| VP78 | 03, K6 |
| V76 | 04, K9 |

Fig. 4 is a graph showing the bactericidal effect of the fermented Stevia extract original fluid on other pathogenic microorganisms. At least two independently isolated strains for all the above-mentioned species were used for the test. Differences in susceptibility to the aged Stevia extract original fluid were negligibly small among strains of the same species, so that only the species were shown in Fig.4.

Vibrio parahemolyticus was grown in Peptone manufactured by Difco Laboratory (pH 7.8) supplemented with 3% NaCl. V. parahemolyticus and Bacillus cereus were efficiently killed by a 10 V/V% or less aged Stevia extract original fluid. A small portion of B. cereus may have survived in spore form. The colony-forming ability of Salmonella enteritidis and Salmonella typhimurium were decreased from approximately 10⁹ cfu/ml to 10² cfu/ml or less when these bacteria were treated with a 20 V/V% aged Stevia extract original fluid at 37 °C for 2 hours. This bactericidal effect is higher than that of Stevia-sensitive E. coli., Yersinia enterocolitica and Staphylococcus aureus were killed by a 30 V/V% aged Stevia extract solution, showing a bactericidal effect relatively similar to that of Stevia-resistant E. coli.

### EXAMPLE 4

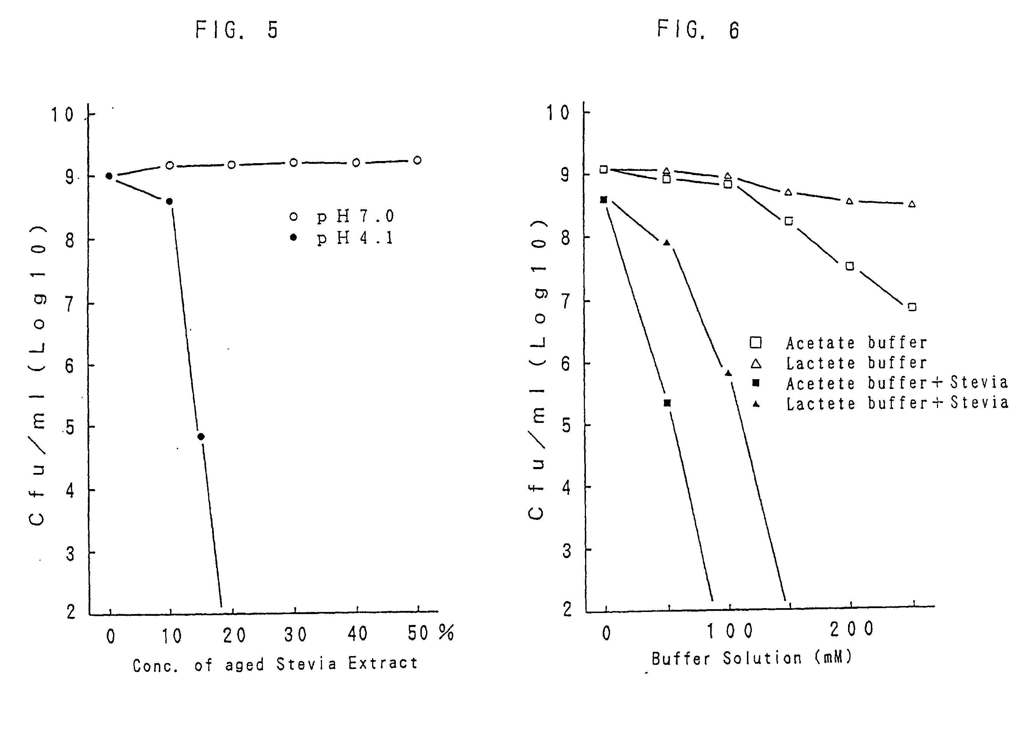

In Fig. 1, the aged Stevia extract original fluid showed highly bactericidal activity towards E. coli EC164 at a concentration of 20 V/V% or more, whereas it lost bactericidal activity when neutralized with 2 N NaOH to pH 7.0 before use. Results thereof are shown in Fig. 5 together with the results shown in Fig. 1. In Fig. 5, the measured pH values were 7.0, 4.7, 4.4, 4.3, 4.3, and 4.2, respectively for the 0, 10, 20, 30, 40, and 50 V/V% solutions of the Stevia extract. The aged Stevia extract original fluid contained acetic acid and lactic acid as major organic acids. However, the bactericidal effect was not obtained under acidic conditions caused by inorganic acids such as hydrochloric acid.

As is apparent from Fig. 5, the pH value of the aged Stevia extract is 4.1, and this acidity takes part in the bactericidal activity. It is therefore understood that the aged Stevia extract shows bactericidal activity under acidic conditions.

### EXAMPLE 5

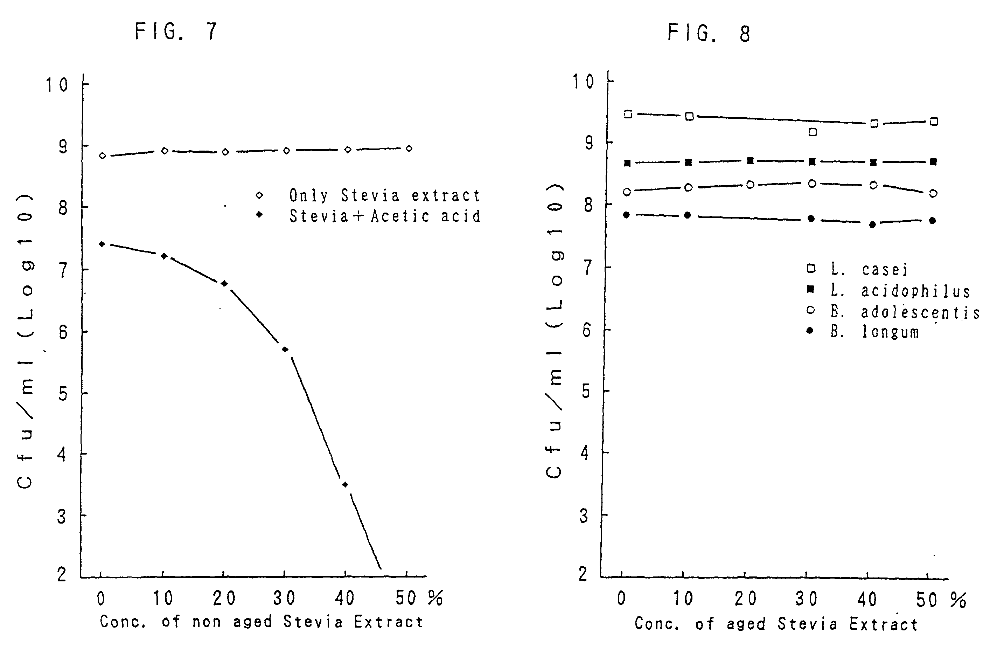

The bactericidal effect of single organic acids was compared with that of mixtures of the organic acids and the Stevia extract to examine the synergetic effect of the organic acids. Results thereof are shown in Fig. 6.

Since the aged Stevia extract original fluid used in this example contained 236 mM lactate and 130 mM acetate, lactate and acetate buffers of pH 4.1 were prepared and the bactericidal effect thereof on E. coli EC164 were examined.

Although the 20 V/V% Stevia extract contained 47 mM lactate and 26 mM acetate, neither the lactate buffer nor the acetate buffer substantially killed E. coli EC164 at a concentration of 100 mM. That is to say, the single acetate and lactate showed bactericidal activity remarkably inferior to that of the aged extract containing the fatty acids of the same concentration as with the 20 V/V% Stevia extract.

However, addition of the 10 V/V% Stevia extract, namely not higher than the effective concentration, to both the fatty acids results in exhibition of high bactericidal activity. From this fact, it is deduced that the bactericidal activity of the aged Stevia extract is obtained and promoted under acidic conditions owing to the organic acids.

### EXAMPLE 6

The bactericidal effect of a non-aged Stevia extract original fluid prepared immediately after extraction and a non-aged Stevia extract original fluid supplemented with acetic acid on E. coli EC164 was assayed. Results thereof are shown in Fig. 7.

The non-aged Stevia extract original fluid used in this example was extracted with hot water according to the extraction method described in "(1) Preparation of Stevia Test Extract", and used as such without fermentation. The non-aged Stevia extract original fluid had a pH of about 7, and showed no bactericidal activity towards E. coli EC164 as shown in Fig. 7. On the other hand, when 150 mM of acetate buffer of pH 4.1 was added, the non-aged Stevia extract original fluid showed the bactericidal effect depending on the amount of the non-aged extract fluid added. From this fact, it is considered that the bactericidal effect of the Stevia extract fluid partly depends on the substances which are present in the Stevia extract before aging.

### EXAMPLE 7

Fig. 8 is a graph showing the bactericidal effect of the aged Stevia extract on Bifidobacteria and Lactobacilli. Bifidobacteria and Lactobacilli used in this example were as follows:
Bifidobacterium longum JCM 1217 (ATCC 15707)
Bifidobacterium adolescentis JCM 1275 (ATCC 15703)
Lactobacillus acidophilus JCM 1275 (ATCC 4356, IFO 13951)
Lactobacillus casei JCM 1134 (ATCC 393)

The bactericidal effect was assayed according to the procedure described in "(3) Assay of Bactericidal Effect". Bifidobacteria were grown in BL medium (manufactured by Nissui Pharmaceutical Co.) and Lactobacilli were grown in MRS medium under anaerobic conditions. As is apparent from Fig. 8, the aged Stevia extract original fluid showed no significant bactericidal effect on B. longum, B. adolescentis, L. acidophilus, and L. casei even at high concentrations of up to 50 V/V%.

This fact means that the bactericide of the present invention does not act on the microorganisms useful for animals and is a very desirable bactericide.

### EXAMPLE 8

Toxicity test was performed, because the bactericide is preferably low in toxicity. However, the bactericide of the present invention exhibits too low toxicity to measure accurate values thereof. That is to say, the aged Stevia extract original fluid was administered to BALB/C mice once per day for a week at high doses of 50 g/kg body weight. However, no abnormality was observed.

## Claims

1. A selective bactericide against food-borne pathogenic bacteria which comprises an extract from plant tissue of Stevia including at least one organic acid, which is added to the extract from plant tissue of Stevia.

2. The selective bactericide according to claim 1, wherein the extract from plant tissue of Stevia is an extract which has been produced with hot water, cold water or a lower alcohol.

3. The selective bactericide according to one of claims 1 and 2, wherein the organic acid or the organic acids is/are selected from acetic acid, lactic acid, propionic acid, valeric acid, citric acid, tartaric acid and malic acid.

4. The selective bactericide against food-borne pathogenic bacteria according to one of claims 1 to 3, wherein the extract from plant tissue of Stevia is fermented.

5. The selective bactericide according to one of claims 1 to 4, which comprises a fermented extract from plant tissue of Stevia and the organic acid(s).

6. Use of a fermented extract from plant tissue of Stevia containing at least one organic acid as a selective bactericide against food-borne pathogenic bacteria.

## Patentansprüche

1. Selektives Bakterizid gegen in Nahrungsmitteln mitgeführten, pathogenen Bakterien, welches ein Extrakt aus dem Pflanzengewebe von Stevia umfaßt, das wenigstens eine organische Säure einschließt, welche zu dem Extrakt aus dem Pflanzengewebe von Stevia zugegeben wurde.

2. Selektives Bakterizid gemäß Anspruch 1, wobei das Extrakt aus dem Pflanzengewebe von Stevia ein Extrakt ist, welches mit heißem Wasser, kaltem Wasser oder einem Niedrigalkohol erzeugt wurde.

3. Selektives Bakterizid gemäß einem der Ansprüche 1 oder 2, wobei die organische Säure oder die organischen Säuren aus Essigsäure, Milchsäure, Propionsäure, Valeriansäure, Zitronensäure, Weinsäure und Malinsäure ausgewählt ist/sind.

4. Selektives Bakterizid gegen in Nahrungsmitteln mitgeführten, pathogenen Bakterien gemäß einem der Ansprüche 1 bis 3, wobei das Extrakt aus dem Pflanzengewebe von Stevia fermentiert ist.

5. Selektives Bakterizid gemäß einem der Ansprüche 1 bis 4, welches einen fermentierten Extrakt aus dem Pflanzengewebe von Stevia und die organische(n) Säure(n) umfaßt.

6. Verwendung eines fermentierten Extrakts aus dem Pflanzengewebe von Stevia, welches wenigstens eine organische Säure enthält, als selektives Bakterizid gegen in Nahrungsmitteln mitgeführte, pathogene Bakterien.

## Revendications

1. Bactéricide sélectif pour l'inhibition de bactéries pathogènes alimentaire, qui comprend un extrait de tissu végétal de Stevia contenant au moins un acide organique, qui est ajouté à l'extrait de tissu végétal de Stevia.

2. Bactéricide sélectif suivant la revendication 1, dans lequel l'extrait de tissu végétal de Stevia est un extrait qui a été produit par utilisation d'eau chaude, d'eau froide ou d'alcool inférieur.

3. Bactéricide sélectif suivant l'une des revendications 1 et 2, dans lequel l'acide organique ou les acides organiques sont choisis parmi l'acide acétique, l'acide lactique, l'acide propionique, l'acide valérique, l'acide citrique, l'acide tartrique et l'acide malique.

4. Bactéricide sélectif contre des bactéries pathogènes alimentaire suivant l'une des revendications 1 à 3, dans lequel l'extrait de tissu végétal de Stevia est fermenté.

5. Bactéricide sélectif suivant l'une des revendications 1 à 4, qui comprend un extrait fermenté de tissu végétal de Stevia et un ou plusieurs acides organiques.

6. Utilisation d'un extrait fermenté de tissu végétal de Stevia contenant au moins un acide organique comme bactéricide sélectif pour l'inhibition de bactéries pathogènes alimentaires.
